# EUROPÄISCHE PATENTSCHRIFT

(11) **EP 2 165 671 B1**
(45) Veröffentlichungstag und Bekanntmachung des Hinweises auf die Patenterteilung: **01.06.2011**
(21) Anmeldenummer: 08164701.8
(22) Anmeldetag: 19.09.2008
(51) Int. Cl.: A61B 19/00, A61B 17/00

(54) **Chirurgisches Pointerinstrument mit Spitzensensor**
Surgical pointer instrument with tip sensor
Instrument chirurgical de pointeur doté d'un capteur de pointe

(43) Veröffentlichungstag der Anmeldung: 24.03.2010
(73) Patentinhaber: BrainLAB AG, 85622 Feldkirchen (DE)
(72) Erfinder: Birkenbach, Rainer, 85435 Erding (DE); Vilsmeier, Stefan, 80539 München (DE)
(74) Vertreter: Schwabe - Sandmair - Marx

(56) Entgegenhaltungen:
- EP-A1- 1 302 172
- WO-A2-2008/104914
- DE-U1- 29 904 018
- US-A1- 2006 142 656
- US-A1- 2007 213 692
- US-A1- 2008 009 697
- US-A1- 2008 200 927
- US-B1- 6 666 875

## Beschreibung

Die Erfindung betrifft ein chirurgisches System gemäß dem Anspruch 1. Pointerinstrumente bzw. Zeigerinstrumente werden speziell im Umfeld einer medizintechnischen Navigation im größeren Umfang verwendet, wobei dies auch für die hierin beschriebenen erfindungsgemäßen Instrumente gilt. Eine spezielle Art der Verwendung besteht in dem Anfahren von Punkten am Patienten (Landmarken) oder an medizintechnischen Hilfseinrichtungen mit der Spitze des Pointerinstruments. Kalibrierte oder vorkalibrierte Pointerinstrumente werden dabei mit Hilfe eines medizintechnischen Trackingsystems (z.B. über Kameras und Positionsmarker am Instrument) lokalisiert; so ist die Position der Spitze des Instruments bekannt und damit auch die Position der Landmarke - oder ganz allgemein - der Stelle, die mit der Pointerspitze angefahren wird.

Einerseits ergibt sich also die Notwendigkeit der Kalibrierung des Instruments vor dem Einsatz bzw. die Notwendigkeit der Verwendung eines vorkalibrierten Instruments, dessen Form und Abmessungen, insbesondere Spitzenposition, für das Navigationssystem bekannt sein müssen. Andererseits ist die Verwendung eines solchen Instruments mit einem Navigationssystem meist noch sehr bedienungsintensiv, weil der Benutzer dem Navigationssystem immer mitteilen muss, dass er gerade eine Landmarke angefahren hat, so dass deren Position aufgenommen bzw. gespeichert werden kann. Wenn mehrere Punkte nacheinander aufgenommen werden oder ein Konturverlauf mit der Pointerspitze kontinuierlich abgefahren wird, können sich Schwierigkeiten ergeben, weil man dem Navigationssystem andauernd mitteilen muss, ob man gerade auf der abzutastenden Kontur liegt oder die Pointerspitze gerade abgehoben und an einen anderen Ort überführt wird. Wenn im letzteren Fall die Navigation nicht rechtzeitig "unterrichtet" wird, werden bei der Überführungsbewegung des Pointers völlig sinnlose Konturdaten aufgenommen.

Ein gattungemäßes System ist aus der EP 1 302 172 A1 bekannt. Die US 2007/02136 92 A1 beschreibt ein chirurgisches Instrument mit einem abnehmbaren Werkzeug und einem fastsensor.

Es ist die Aufgabe der vorliegenden Erfindung, ein chirurgisches System bereitzustellen, welches die Handhabung mit einem medizintechnischen Navigationssystem einfach und bedienerfreundlicher macht.

Diese Aufgabe wird erfindungsgemäß durch ein chirurgisches System gemäß dem Anspruch 1 gelöst. Die Unteransprüche definieren bevorzugte Ausführungsformen der Erfindung.

Gemäß der vorliegenden Erfindung wird also ein chirurgisches System bereitgestellt, das einen Griffabschnitt und eine Spitze sowie einen Spitzensensor am Griffabschnitt umfasst. Der Spitzensensor heißt deshalb so, weil er verschiedenste Merkmale detektiert, die mit der Spitze des Instruments verbunden sind und die später noch im Einzelnen erläutert werden. Mit anderen Worten ist der Griffabschnitt des erfindungsgemäßen Instruments derart ausgestaltet, dass er Informationen über die Instrumentenspitze detektieren kann, und gerade diese Eigenschaft des erfindungsgemäßen Instruments macht seine Handhabung sehr viel einfacher. Verschiedenste Informationen über die Spitze und/oder ihre Zusammenwirkung oder Wechselwirkung mit dem Griff können nämlich verwendet werden, um die Navigation einfacher, unaufwändiger und bedienerfreundlicher zu machen, weil solche Informationen (z.B. über die Art oder die momentane Belastung der Spitze) Informationseingaben ersetzen können, die ansonsten durch den Benutzer am Navigationssystem vorgenommen werden müssen.

Gemäß der vorliegenden Erfindung ist mindestens eine Spitze abnehmbar am Griffabschnitt befestigbar, insbesondere mit einer lösbaren Befestigung, speziell einer Steckbefestigung. Es können mehrere Spitzen wechselbar am Griffabschnitt befestigt werden, speziell mittels eines für mehrere oder alle Spitzen gleichen Befestigungsadapters, der eine einfache Befestigung und damit auch einen einfachen Spitzenwechsel gestattet. Nach der Befestigung sollte die Spitze starr und ohne Spiel am Griff sitzen.

Die Spitze kann einen mit dem Spitzensensor komplementär zusammenwirkenden Griffbefestigungsabschnitt (kann auch der Befestigungsadapter sein) aufweisen, und gemäß einer Ausführungsvariante der vorliegenden Erfindung umfasst der Spitzensensor einen Präsenzsensor für das Vorhandensein einer Spitze. Der Präsenzsensor wird insbesondere automatisch arbeiten, also ohne weitere Betätigung, d.h. ohne Benutzereingriff, das Vorhandensein einer Spitze feststellen.

Es ist aber im Rahmen der vorliegenden Erfindung auch möglich, dass nicht lediglich das Vorhandensein einer Spitze festgestellt wird, sondern dass Spitze und Griff oder eines der beiden Elemente eine Spitzenidentifizierungsvorrichtung umfassen, insbesondere mit einer Erkennungsvorrichtung bzw. Auslesevorrichtung für die Art bzw. die Eigenschaften der Spitze, wobei die Vorrichtung dem Spitzensensor zugeordnet ist, und mit einem Erkennungsmerkmal an der Spitze.

Die Spitzenidentifizierungsvorrichtung kann eine Formidentifizierung bzw. einen Formschlüssel (z.B. in der Art eines Türschlüssels), eine RFID-Identifizierung und/oder eine Strichcode-Identifizierung umfassen. Es besteht die Möglichkeit, das Instrument so auszugestalten, dass der Spitzenabschnitt, der mit dem Spitzensensor in Wechselwirkung tritt, speziell der Griffbefestigungsabschnitt mit dem Erkennungsmerkmal Informationen über die Art bzw. die Eigenschaften der Spitze trägt.

Gemäß einem der vorliegenden Erfindung umfasst der Spitzensensor einen Kraftsensor für die von einer Spitze auf dem Griffabschnitt ausgeübte Kraft. Mit diesem Kraftsensor kann erfasst werden, ob die Spitze gerade gegen einen Widerstand drückt und das Instrument deshalb momentan einsatzbereit oder im Einsatz ist. Beispielsweise lässt sich so feststellen, ob gerade eine Landmarke zur Positionserfassung angefahren wird. Auch gestattet die Krafterfassung eine Detektion über längere Zeit, so dass festgestellt werden kann, ob beispielsweise eine Pointerspitze zur Konturerfassung über eine Oberfläche gleitet. Die Konturerfassung würde dann nur solange stattfinden, wie eine Kraft von der Spitze auf den Griff gemessen wird. Beim Abnehmen des Instruments von der Oberfläche wird auch keine Kraft mehr ausgeübt, und dies kann die Konturerfassung im Navigationssystem unmittelbar und automatisch ausschalten.

Allgemein ausgedrückt ist bei dem chirurgischen System gemäß der vorliegenden Erfindung eine funktionelle Interaktionsvorrichtung vorgesehen, welche Daten über die Zusammenwirkung von Spitze und Griffabschnitt zur Unterstützung einer bildgeführten Chirurgie an ein medizintechnisches Navigationssystem übermittelt. Dabei weist die funktionelle Interaktionsvorrichtung einen Datenkommunikator auf, insbesondere einen Funküberträger, wie einen Bluetooth- oder Hochfrequenz- bzw. Radiofrequenz-Kommunikator/Sender oder einen Infrarot-, Schall- bzw. Ultraschall-Kommunikator/Sender. Natürlich kann bei einem erfindungsgemäß angesetzten System auch ein Empfänger für diese kommunizierten Daten bereitgestellt werden, speziell ein mit dem medizintechnischen Navigationssystem verbundener Empfänger für die Daten des Datenkommunikators.

Das Instrument kann eine elektrische Energieversorgung, speziell mit einem Energiespeicher (Batterie, Akkumulator) und/oder eine Energieerzeugung umfassen. Der Akkumulator wird bei einer Ausführungsform speziell berührungslos bzw. durch Induktionsaufladung aufladbar sein. Was die Energieerzeugung betrifft, so kann diese eine oder mehrere der folgenden Vorrichtungen umfassen:
- eine Piezo-Energieerzeugung;
- eine thermoelekrische Energieumwandlung;
- eine Solarzellen-Energieumwandlung.

Im Folgenden - bis zur Beschreibung der Figuren - werden noch weitere allgemeine Informationen über mögliche Ausführungsformen eines erfindungsgemäßen chirurgischen Instruments, speziell eines Pointerinstruments für die bildgestützte Chirurgie gegeben.

Was den Griff betrifft, so kann dieser mit Navigationsmarkern ausgestattet sein, wobei speziell zwei oder mehr reflektive Kugelmarker vorgesehen werden können. Die Geometrie der Kugelmarker-Anordnung wird von der maximalen Instrumentenspitzen-Länge abhängen. Alternativ zu Kugelmarkern können auch flache, z. B. kreisrunde, reflektive Schichten verwendet werden.

Wie schon erwähnt ist die Spitzen-Schnittstelle vorzugsweise so ausgeführt, dass sie auswechselbare Spitzen haltern kann, die leicht wechselbar und starr bzw. ohne Spiel befestigt sein sollten. Was die Spitzen-Detektion angeht, können verbundene Spitzen vorzugsweise automatisch detektiert werden. Dies kann mit Hilfe eines Schlüsselmechanismus geschehen, d.h. der Befestigungsmechanismus ist wie ein Auto- oder Hausschlüssel ausgestaltet, der eine Identifikation aus Geometriedaten und Fixierung mit einer Vorrichtung umfasst. An der Spitzenseite kann beispielsweise die Schlüsselform vorhanden sein, während der Griff wie ein Schloss ausgebildet ist. Natürlich ist eine umgekehrte Anordnung grundsätzlich möglich.

Wenn zur Identifizierung eine RFID-Technik verwendet wird, kann an der Spitze ein energiefreier RFID-Chip vorhanden sein, während sich der RFID-Leser im Griff befindet. Alternativ könnte der Leser sich auch in der Navigations-Station befinden. Wenn ein Strichcode (Barcode) als Identifikationsmittel verwendet wird, kann dieser an der Spitzenschnittstelle angeformt sein (Spritzguss) oder durch Lasergravur ausgebildet werden. Natürlich ist auch ein einfacher aber haltbarer Aufdruck denkbar. Im Griff befände sich dann ein einfaches optisches Lesegerät, das sequenziell (Code wird beim Eingleiten der Spitze gelesen), komplett (Code wird im Ganzen gelesen) oder mit einer Ferndetektion arbeitet, bei welcher der Code durch das Navigations- bzw. Trackingsystem gelesen wird. Hierzu kann am Navigations- bzw. Trackingsystem eine eigene Lesevorrichtung angebracht werden - oder man verwendet die Tracking-Kamera, um den Code zu lesen.

Der Status der mit dem Griff verbundenen Instrumentenspitze kann regelmäßig über eine Kommunikationsvorrichtung abgesendet werden, beispielsweise einmal pro Sekunde, und verschiedene Status-Informationen könnten sein: "keine Spitze vorhanden", "Spitze entfernt", "Spitze eingesetzt", "unbekannte Spitze", "Spitze erfasst" oder "Spitze erfasst und OK".

Es können eine gewisse Anzahl von Spitzen identifiziert werden, und hierfür ist es von Vorteil, die geometrischen Spitzendaten zu speichern. Sinnvollerweise werden die Spitzendaten entweder in bzw. an der Spitze selbst gespeichert, bzw. bereitgestellt oder im Navigationssystem. Im ersten Fall können die Daten codiert in einer Identifizierungsvorrichtung vorhanden sein. So kann der Schlüssel sowohl die Identifizierung als auch die Spitzendaten als Forminformation umfassen, und diese Daten lassen sich auch in Strichcodes oder RFIDs speichern. Es besteht auch die Möglichkeit, alle Daten oder auch einen Teil der Daten im Navigationssystem unterzubringen, wobei beispielsweise der Griff feststellt, welche Spitze angebracht wird und dann die genaueren Daten über die Spitze im Navigationssystem vorhanden sind.

Als Datenformat kann eine Grundinformation gewählt werden, die eine mechanische Beschreibung der Form (Vektorgraphik, Splines etc.) umfasst, oder eine vollständige 3D-Formbeschieibung (XML, VFML, STML). Ein Spitzenwechsel kann - wie schon erwähnt - automatisch weitergemeldet werden (Spitzendetektion, Kommunikationsvorrichtung). Es ist möglich, eine gebogene Spitze einfach durch eine Überprüfung mit der Navigationssoftware oder durch ein Halten, Schwenken an einer Oberfläche zu identifizieren, wenn nicht - was erfindungsgemäß möglich ist - alle geometrischen Daten z. B. in der Software hinterlegt sind.

Es wird ein Kraftsensor verwendet welcher die Kraft müsst, die auf die Spitze ausgeübt wird, indem er die Kraft misst, welche von der Spitze auf den Griffabschnitt übergeht. Es besteht die Möglichkeit, den Druck bzw. die Kraft konstant auf einem Navigationsbildschirm oder auch als LED-Anzeige (z.B. als Leuchtstreifen auf dem Griff) anzuzeigen, vorteilhafterweise mit einem grün-gelb-rot-Anzeigelauf, der unkritische, kritische und übermäßige Belastungen zeigt. Diese Kraft- bzw. Druckmessung wird auch als ein "Schalter" verwendet, beispielsweise wenn Oberflächen-Matching-Tätigkeiten durchgeführt werden und man in jedem Moment wissen muss, ob die Spitze des Instruments etwas berührt. Bei Hautberührungen würde die Schwelle für die Kraft, die eine Berührung anzeigt, niedrig liegen, bei Knochen-Abtastungen läge diese Schwelle höher. Außerdem kann man durch eine solche Kraftmessung feststellen, ob eine Punkt-Akquirierung gerade auf einem Knochen oder auf einem Weichgewebe (Muskel, Haut) durchgeführt wird.

Als Material für die Instrumentenspitze kommen Metall oder Kunststoffe in Frage; die Spitzen können wegwerfbar sein. Die Schnittstelle kann als Standard-Schnittstelle für mehrere Griffe oder Griffarten ausgestaltet werden.

Was die Kommunikation des Instruments mit dem Navigationssystem (IGS-System = Image Guided Surgery System) betrifft, so kann diese Kommunikation unidirektional sein, d.h. bei angesetztem Griff werden Sensordaten abgesendet. Die Kommunikation sollte möglichst wenig so genannte Line-of-Sight-Probleme haben, was bei einer Funk-Kommunikation gesichert wäre. Denkbar ist aber auch eine Schall-Kommunikation oder eine Infrarot-Kommunikation, bei der als Empfänger eine Tracking-Kamera des Navigationssystems oder ein separater Empfänger verwendet werden. Auch eine bidirektionale Kommunikation ist möglich, bei der z.B. vom IGS-System ermittelte Fehler durch LEDs am Griff angezeigt werden.

Am Griff können verschiedene Knöpfe zur Steuerung der Navigationssoftware angebracht werden, nämlich z.B. Druckknöpfe, Kippknöpfe oder Drehknöpfe (eins bis drei, speziell zwei bis drei Knöpfe). Sie können mechanischer aber auch elektromechanischer Natur oder Sensorknöpfe sein. Damit sie leicht mit chirurgischen Handschuhen bedient werden können, können sie ein abfühlbares Muster auf ihrer Oberfläche haben.

Energie wird beispielsweise für die Kommunikation, für den Sensor und die Knöpfe benötigt, und es können Batterien bereitgestellt werden, die vom Griff umschlossen sind, mitsterilisiert werden können und eine Lebensdauer von einigen Jahren aufweisen. Auch Austauschbatterien sind denkbar, ebenso wie wieder aufladbare Akkumulatoren oder thermoelektrische Elemente, welche Wärmeenergie zu elektrischer Energie umwandeln. Insbesondere könnte dann die Energie beim Sterilisierungsprozess zur Aufladung der Elektro-Energieversorgung genutzt werden. Außerdem ist eine Energiegewinnung durch Piezo-Technik möglich, wobei das Drücken eines Knopfes genügend Energie erzeugen könnte, um beispielsweise die Kommunikation zu unterstützen.

Die Erfindung wird im Weiteren anhand verschiedener Ausführungsformen und mit Hilfe der beiliegenden Zeichnungen näher erläutert. Sie kann alle hierin beschriebenen Merkmale einzeln sowie in jedweder sinnvollen Kombination umfassen. Insbesondere kann sie auch spezielle Verwendungen der gezeigten Vorrichtungen oder mit ihnen durchgeführte und hier aufgezeigte Verfahren umfassen. In den Zeichnungen zeigen:
- Figur 1: eine schematische Darstellung eines erfindungsgemäßen Instruments mit mehreren wechselbaren Spitzen;
- Figur 2: ein Schema für eine induktive Energieaufladung; und
- Figur 3: ein Schema für eine thermoelektrische Energieaufladung des Instruments.

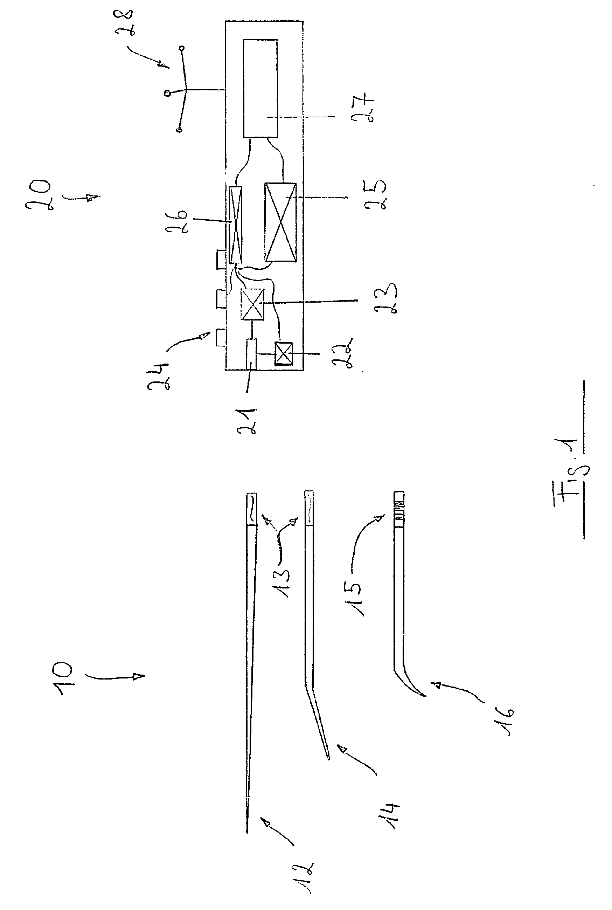

In Figur 1 sind verschiedene Alternativen eines chirurgischen Instruments gezeigt, nämlich mit unterschiedlichen Spitzen 12, 14, 16 (wobei insgesamt auch das Bezugszeichen 10 allgemein für die Spitze verwendet wird) und einem Griffabschnitt 20. Bei den Spitzen handelt es sich um verschiedene Spitzen für einen chirurgischen Pointer, wobei die Spitze 12 eine lange gerade Spitze, die Spitze 14 eine Spitze mit abgeknicktem Vorderteil und die Spitze 16 eine abgerundete Spitze ist. Alle Spitzen haben einen Befestigungsabschnitt an dem Ende, das ihrem vorderen Ende gegenüberliegt, und bei den Spitzen 12 und 14 ist dieser Befestigungsabschnitt 13 als Schlüsselform-Abschnitt ausgebildet. Die Instrumentenspitze 16 hat einen Befestigungsabschnitt 15, der mit einem eingravierten oder aufgedruckten Strichcode versehen worden ist. Die Befestigungsabschnitte 13, 15 dienen einerseits der Befestigung der Spitze am Griff, andererseits auch ihrer Identifizierung und der Bereitstellung von Daten, nämlich Formdaten, über die Spitze. So kann der Schlüsselabschnitt 13 durch mechanische Formgebung diese Daten aufweisen, während sie im Strichcode des Abschnittes 15 verschlüsselt untergebracht sind.

Mit dem Befestigungsabschnitt 13, 15 (Adapter) wird die Spitze 10 mit dem Griff 20 verbunden, der hier eher schematisch und (zur Vereinfachung der Vorstellung) in seinen Abmessungen etwas verzerrt dargestellt ist. Die Spitzen 10 fahren mit den Befestigungsabschnitten in die Spitzenaufnahme 21 ein und werden dort befestigt. Ein erster Sensor 22 ist der Spitzenaufnahme 21 zugeordnet, und der Sensor 22 kann einerseits das Vorhandensein einer Spitze detektieren, andererseits bei speziellen Ausführungsformen auch eine Identifikation der Spitze über die Informationen ermöglichen, die durch die Befestigungsabschnitte 13, 15 bereitgestellt werden. Wenn die Aufnahme 21 komplementär als ein Schloss für die Schlüsselform 13 ausgebildet ist, wird eine mechanische Abtastung sowohl das Vorhandensein der Spitze detektieren als auch die zusätzlichen Informationen abrufen können. Im Falle der Spitze 16 kann der Sensor 22 einen Strichcodeleser umfassen.

Ein weiteres Bauelement im Griff 20 ist der Kraftsensor 23. Mit ihm wird über die Kraft, die von der Spitze auf den Sensor 23 ausgeübt wird, wiederum diejenige Kraft bzw. derjenige Druck festgestellt, die bzw. der auf das vordere Ende der jeweiligen Spitze ausgeübt wird. Wie schon beschrieben, wird diese Kraftinformation bzw. Druckinformation in verschiedener Weise ausgewertet, nämlich als "Schalter" für die Punkt- oder Flächenakquirierung und als Belastungsinformation, wobei man unterscheiden kann, ob der Pointer auf weiches oder hartes Gewebe trifft.

Das Bezugszeichen 25 steht hier für eine weitere Vorrichtung, die am Griff angebracht werden kann, beispielsweise eine Induktionszelle oder eine Solarzelle zur Energiegewinnung, oder auch eine Anzeige (LED-Anzeige) für die Druckwerte, die mit dem Sensor 23 gemessen werden. Das Bezugszeichen 27 deutet auf eine Energieversorgung, d.h. eine Batterie oder einen Akkumulator, der mit den Energie erzeugenden oder Energie verbrauchenden Elementen im Griff verbunden ist. Stellvertretend ist z.B. eine Verbindung mit dem Element 25 gezeigt, das als Solarzelle oder als Induktionszelle bzw. Piezo-Zelle Strom an die Energieversorgung 27 liefern kann.

Damit Informationen bzw. Daten vom Griff weitergegeben werden können, weist dieser einen Datenkommunikator oder Funküberträger 26 auf. Es ist angedeutet, dass alle Elemente bzw. Sensoren 22, 23, 25 sowie die bereitgestellten Knöpfe 24 (zur Steuerung der Navigationssoftware) mit diesem Datenkommunikator bzw. Funküberträger 26 verbunden sind und ihm Daten liefern, die er an ein Navigationssystem weitergeben kann. Natürlich verbraucht der Überträger 26 dabei Energie und ist deshalb ebenso mit der Batterie bzw. mit dem Akkumulator 27 verbunden. Übertragene Daten sind Druck- bzw. Kraftdaten des Sensors 23 und können Identifizierungsdaten für die Spitzen sein, aber auch Befehle, die über die Knöpfe 24 eingegeben werden oder Statusdaten (Akkufüllstand). Das Instrument kann ein navigiertes Instrument sein, wie aus der Referenzanordnung 28 hervorgeht, die am Griff befestigt ist und drei reflektive Kugelmarker aufweist.

Bei einem einfachen Anwendungsfall wird die Spitze 12 mit dem Befestigungsabschnitt 13 in die Aufnahme 21 des Griffs 20 eingesteckt. Über die Schlüsselinformation im Befestigungsabschnitt 13, die durch den Sensor 22 ausgelesen wird, wird die Spitze 12 identifiziert. Entweder stehen schon durch die Informationen im Befestigungsabschnitt 13 die Daten über die Länge der Spitze und ihre Form zur Verfügung oder diese Daten sind im Navigationssystem gespeichert. Mit diesen Informationen über die Spitze 12 steht sozusagen ein "vorkalibriertes" Instrument aus Spitze 12 und Griff 20 zur Verfügung, das unmittelbar vom Navigationssystem positionell erfasst und verwendet werden kann, beispielsweise zur Akquirierung von Landmarken an einem Patienten. Bei einer solchen Akquirierung wird der Druck auf die Spitze mit dem Sensor 23 überwacht werden. Falls eine Landmarke auf der Haut des Patienten akquiriert, d.h. positionell durch die Pointerspitze erfasst werden soll, wird nur ein geringer Druck mit der Pointerspitze notwendig sein. Wenn zu fest mit der Pointerspitze auf die Landmarke gedrückt wird, die sich auf der Haut befindet, könnte sich die Landmarke (Haut) verschieben und somit das Navigationsergebnis verfälscht werden. Diesen Druck aber wird man mit dem Sensor 23 überwachen, so dass entsprechende Fehlakquirierungen vermieden werden können. Beispielsweise wird mit dem Pointer dann nur so auf die Landmarke gedrückt, dass eine Leuchtstreifen-Anzeige 25 (hier als Ausführungsform) einen grünen Wert anzeigt.

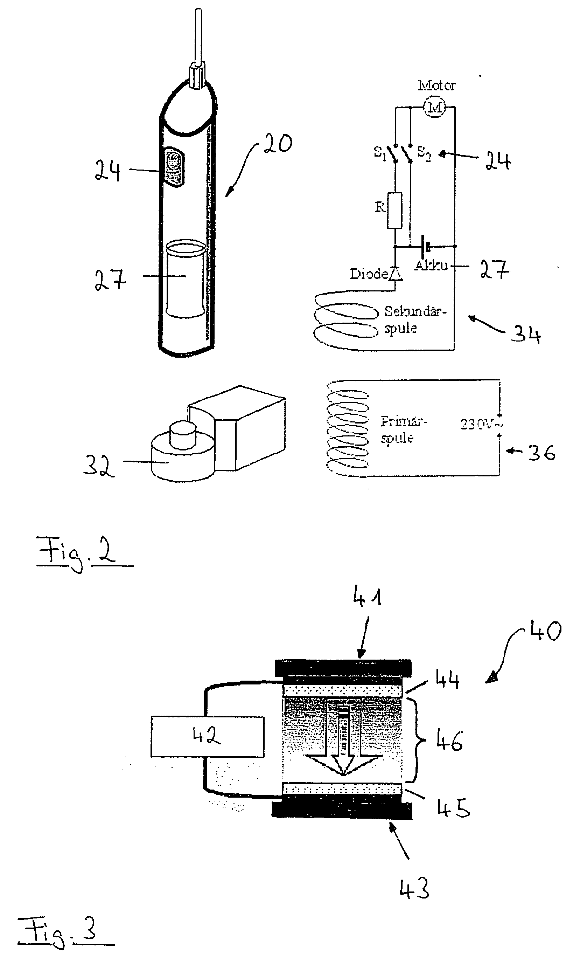

Eine Energieversorgung bzw. ein Wiederaufladen eines Akkus kann so erfolgen, wie es beispielsweise in Figur 2 dargestellt ist. Das Instrument 20 hat einen Akku 27, der eingeschweißt und dicht verschlossen ist, so dass das Instrument ohne Weiteres sterilisiert werden kann. Der Griff 20 des Instruments wird dazu auf die Ladestation 32 aufgesetzt, und die Schaltungen rechts zeigen, dass die Ladestation einen Primärkreis 36 mit einer Primärspule aufweist, welche induktiv den Sekundärkreis 34 bedient und über die Sekundärspule den Akku 27 auflädt. Eine solche Ausführungsform mit eingeschlossenem Akku 27 ist sterilisationstechnisch sehr vorteilhaft.

Eine andere Form der Energieversorgung ist aber auch denkbar, nämlich eine thermoelektrische Energiegewinnung, wie sie beispielsweise und schematisch in der Figur 3 dargestellt ist. Um eine elektrische Last 42 zu bedienen, wird hierzu das thermoelektrische Element 40 bereitgestellt, das eine Wärmequelle 41 und eine Kältesenke 43 aufweist. Die Wärmequelle könnte beispielsweise die Sterilisationswärme sein, während die Kältesenke durch das anfänglich noch kalte Instrument bei der Sterilisation zur Verfügung gestellt wird. An die Wärmequelle schließt sich ein so genannter Solid State Emitter 44 an, und zwischen diesem Solid State Emitter 44 und einem gegenüberliegenden Solid State Collector 45 befindet sich ein thermoelektrischer Halbleiter. Solange ein Temperaturgradient zwischen der Wärmequelle 41 und der Kältesenke 43 vorhanden ist, kann die elektrische Last 42 bedient, beispielsweise der Akku 27 aufgeladen werden.

Eine solche Energieerzeugung könnte man beispielsweise in den Instrumentengriff einbauen, wobei die Wärmequelle außen gelegen wäre und die Kältesenke innen oder mittig, wo das Instrument sich am längsten kühl hält.

## Patentansprüche

1. System, umfassend ein medizintechnisches Navigationssystem und ein Pointerinstrument mit einem Griffabschnitt (20) und einer Pointerspitze (10), wobei mindestens eine Spitze (10) abnehmbar am Griffabschnitt (20) befestigbar ist und am Griffabschnitt (20) ein die auf die Pointerspitze (10) ausgeübte Kraft messender Spitzensensor (22, 23) vorgesehen ist, mittels dem in jedem Moment erfasst werden kann, ob die Spitze (10) des Instruments gerade etwas berührt, wobei eine funktionelle Interaktionsvorrichtung vorgesehen ist, welche ausgebildet ist, um Daten über die Zusammenwirkung von Spitze (10) und Griffabschnitt (20) zur Unterstützung einer bildgeführten Chirurgie an das medizintechnische Navigationssystem zu übermitteln, wobei die funktionelle Interaktionsvorrichtung einen Datenkommunikator aufweist, der ausgebildet ist, um Belastungsinformationen über die Höhe der von der Spitze (10) auf den Griffabschnitt (20) übergehenden Kraft weiterzugeben, **dadurch gekennzeichnet, dass** das System so ausgebildet ist, dass die Kraftinformation auch als Schalter verwendet wird, um festzustellen, ob die Spitze des Instruments etwas berührt.

2. System nach Anspruch 1, **dadurch gekennzeichnet, dass** mindestens eine Spitze (10) abnehmbar am Griffabschnitt (20) mittels einer Steckbefestigung befestigbar ist.

3. System nach Anspruch 1 oder 2, **dadurch gekennzeichnet, dass** mehrere Spitzen (10) wechselbar am Griffabschnitt (20) befestigt werden können, speziell mittels eines für mehrere oder alle Spitzen (10) gleichen Befestigungsadapters (13, 21; 15, 21).

4. System nach einem der Ansprüche 1 bis 3, **dadurch gekennzeichnet, dass** der Spitzensensor einen Präsenzsensor (22) für das Vorhandensein einer Spitze (10) umfasst.

5. System nach einem der Ansprüche 1 bis 4, **dadurch gekennzeichnet, dass** die Spitze (10) und der Griff eine Spitzenidentifizierungsvorrichtung umfassen, insbesondere mit
- einer Erkennungsvorrichtung bzw. Auslesevorrichtung (22) für die Art bzw. die Eigenschaften der Spitze (12, 14, 16), die dem Spitzensensor zugeordnet ist, und
- einem Erkennungsmerkmal (13, 15) an der Spitze (10).

6. System nach Anspruch 5, **dadurch gekennzeichnet, dass** die Spitzenidentifizierungsvorrichtung eine Formidentifizierung bzw. einen Formschlüssel (13), eine RFID-Identifizierung und/oder eine Strichcode-Identifizierung (15) umfasst.

7. System nach einem der Ansprüche 1 bis 6, **dadurch gekennzeichnet, dass** der Spitzenabschnitt (13, 15), der mit dem Spitzensensor (22) in Wechselwirkung tritt, speziell der Griffbefestigungsabschnitt (13, 15) mit dem Erkennungsmerkmal, Informationen über die Art bzw. Eigenschaften der Spitze trägt.

8. System nach Anspruch 1, **dadurch gekennzeichnet, dass** der Datenkommunikator einen Funküberträger, wie einen Bluetooth- oder Hochfrequenz- bzw. Radiofrequenz-Kommunikator/Sender oder einen Infrarot-, Schall- bzw. Ultraschall-Kommunikator/Sender umfasst.

9. System nach einem der Ansprüche 1 bis 8, **dadurch gekennzeichnet, dass** das Pointerinstrument eine elektrische Energieversorgung, speziell mit einem Energiespeicher und/oder einer Energieerzeugung umfasst.

10. System nach Anspruch 9, **dadurch gekennzeichnet, dass** die Energieversorgung eine Batterie oder einen Akkumulator aufweist, der speziell berührungslos bzw. durch Induktionsaufladung aufladbar ist.

11. System nach Anspruch 9, **dadurch gekennzeichnet, dass** die Energieversorgung eine oder mehrere der folgenden Vorrichtungen umfasst:
- eine Piezo-Energieerzeugung;
- eine thermoelektrische Energieumwandlung;
- eine Solarzellen-Energieumwandlung.

## Claims

1. A surgical system, comprising a medical navigation system and a pointer instrument comprising a handle portion (20) and a pointer tip (10), wherein at least one tip (10) can be removably fastened to the handle portion (20), and a tip sensor (22, 23) which measures the force exerted on the pointer tip (10) is provided on the handle portion (20), wherein it is possible by means of the tip sensor (22, 23) to detect at each moment whether the tip (10) of the instrument is currently contacting something, wherein a functional interaction device is provided which is designed to transmit data concerning the co-operation of the tip (10) and the handle portion (20) to the medical navigation system in order to assist in image-guided surgery, wherein the functional interaction device comprises a data communicator which is designed to relay load information concerning the level of the force passing from the tip (10) to the handle portion (20), **characterised in that** the system is designed such that the force information is also used as a switch in order to determine whether the tip of the instrument is contacting something.

2. The system according to claim 1, **characterised in that** at least one tip (10) can be removably fastened to the handle portion (20) by means of a plug fastening.

3. The system according to claim 1 or 2, **characterised in that** a number of tips (10) can be exchangeably fastened to the handle portion (20), specifically by means of a fastening adaptor (13, 21; 15, 21) which is the same for a number of tips or for all the tips (10).

4. The system according to any one of claims 1 to 3, **characterised in that** the tip sensor comprises a presence sensor (22) for the presence of a tip (10).

5. The system according to any one of claims 1 to 4, **characterised in that** the tip (10) and the handle comprise a tip identifying device, in particular comprising:
- a recognition device and/or reading-off device (22) for the type and/or characteristics of the tip (12, 14, 16), which is assigned to the tip sensor; and
- a recognition feature (13, 15) on the tip (10).

6. The system according to claim 5, **characterised in that** the tip identifying device comprises a shape identification and/or shape key (13), an RFID identification and/or a barcode identification (15).

7. The system according to any one of claims 1 to 6, **characterised in that** the tip portion (13, 15) which interacts with the tip sensor (22), specifically the handle fastening portion (13, 15) comprising the recognition feature, bears information concerning the type and/or characteristics of the tip.

8. The system according to claim 1, **characterised in that** the data communicator comprises a radio transmitter such as a Bluetooth or high-frequency and/or radio-frequency communicator/transmitter or an infrared, sonic and/or ultrasonic communicator/transmitter.

9. The system according to any one of claims 1 to 8, **characterised in that** the pointer instrument comprises an electrical energy supply, specifically comprising an energy store and/or an energy generator.

10. The system according to claim 9, **characterised in that** the energy supply comprises a battery or a rechargeable battery which can specifically be charged in a non-contact process and/or by inductive charging.

11. The system according to claim 9, **characterised in that** the energy supply comprises one or more of the following devices:
- a piezoelectric energy generator;
- a thermo-electric energy converter;
- a solar cell energy converter.

## Revendications

1. Système chirurgical, comportant un système de navigation médico-technique et un instrument pointeur avec une partie de poignée (20) et un embout de pointeur (10), dans lequel au moins un embout (10) peut être fixé de manière amovible sur la partie de poignée (20) et est muni d'un capteur d'embout (22, 23) qui mesure la force exercée sur l'embout de pointeur (10), capteur au moyen duquel il est possible de détecter à chaque instant si l'embout (10) de l'instrument touche directement quelque chose, dans lequel est prévu un dispositif fonctionnel d'interaction qui est conçu pour transmettre au système de navigation médico-technique des données concernant la coopération de l'embout (10) et de la partie de poignée (20) afin de faciliter une chirurgie guidée par image, dans lequel le dispositif fonctionnel d'interaction comporte un dispositif de communication de données qui est conçu pour retransmettre des informations de charge concernant la grandeur de la force transférée de l'embout (10) à la partie de poignée (20), **caractérisé en ce que** le système est conçu de telle sorte que les informations de force sont également utilisées comme un commutateur afin de déterminer si l'embout de l'instrument est en contact avec quelque chose.

2. Système selon la revendication 1, **caractérisé en ce qu'**au moins un embout (10) peut être fixé de manière amovible sur la partie de poignée (20) au moyen d'une fixation enfichable.

3. Système selon la revendication 1 ou 2, **caractérisé en ce que** plusieurs embouts (10) peuvent être fixés de manière échangeable sur la partie de poignée (20), en particulier au moyen d'un adaptateur de fixation identique (13, 21 ; 15, 21) pour plusieurs ou la totalité des embouts (10).

4. Système selon l'une quelconque des revendications 1 à 3, **caractérisé en ce que** le capteur d'embout comporte un capteur de présence (22) pour la présence d' un embout (10).

5. Système selon l'une quelconque des revendications 1 à 4, **caractérisé en ce que** l'embout (10) et la poignée comportent un dispositif d'identification d'embout, en particulier avec :
- un dispositif de reconnaissance ou un dispositif de lecture (22) pour le type ou les caractéristiques de l'embout (12, 14, 16) qui est associé au capteur d'embout, et
- une caractéristique de reconnaissance (13, 15) sur l'embout (10).

6. Système selon la revendication 5, **caractérisé en ce que** le dispositif d'identification d'embout inclut une identification de forme ou une clé de forme (13), une identification RFID et/ou une identification par code à barres (15).

7. Système selon l'une quelconque des revendications 1 à 6, **caractérisé en ce que** la partie d'embout (13, 15) qui interagit avec le capteur d'embout (22), en particulier la partie de fixation de poignée (13, 15) avec la caractéristique de reconnaissance, porte des informations concernant le type ou les propriétés de l'embout.

8. Système selon la revendication 1, **caractérisé en ce que** le dispositif de communication de données inclut un transmetteur radio, tel qu'un dispositif de communication/émetteur Bluetooth ou haute fréquence ou radiofréquence, ou un dispositif de communication/émetteur infrarouge, sonique ou ultrasonore.

9. Système selon l'une quelconque des revendications 1 à 8, **caractérisé en ce que** l'instrument pointeur inclut une alimentation en énergie électrique, en particulier avec un accumulateur d'énergie et/ou un générateur d'énergie.

10. Système selon la revendication 9, **caractérisé en ce que** l'alimentation en énergie comporte une batterie ou un accumulateur qui peut être chargé en particulier sans contact ou par une charge inductive.

11. Système selon la revendication 9, **caractérisé en ce que** l'alimentation en énergie inclut un ou plusieurs des dispositifs suivants :
- un générateur d'énergie piézoélectrique,
- un convertisseur d'énergie thermoélectrique,
- un convertisseur d'énergie de cellule solaire.
